(19) Europäisches Patentamt
European Patent Office
Office européen des brevets

(11) **EP 4 458 397 A1**

(12) **EUROPÄISCHE PATENTANMELDUNG**

(43) Veröffentlichungstag:
**06.11.2024   Patentblatt 2024/45**

(21) Anmeldenummer: **24170740.5**

(22) Anmeldetag: **17.04.2024**

(51) Internationale Patentklassifikation (IPC):
*A61M 16/08* (2006.01)     *F16L 11/04* (2006.01)

(52) Gemeinsame Patentklassifikation (CPC):
**A61M 16/0875; F16L 11/112;** A61M 2205/0216;
A61M 2207/00

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH MA MD TN**

(30) Priorität: **05.05.2023   DE 102023001816**
**24.05.2023   DE 102023002102**

(71) Anmelder: **Löwenstein Medical Technology S.A.**
**2557 Luxembourg (LU)**

(72) Erfinder: **SCHULZE, Willy**
**90552 Röthenbach an der Pegnitz (DE)**

(74) Vertreter: **Marx, Thomas**
**Löwenstein Medical Technology GmbH + Co. KG**
**IP Management**
**Kronsaalsweg 40**
**22525 Hamburg (DE)**

(54) **BEATMUNGSSCHLAUCH UND VERFAHREN ZUR HERSTELLUNG EINES BEATMUNGSSCHLAUCHES**

(57)    Die Erfindung umfasst ein Verfahren zur Herstellung eines Beatmungsschlauches und einen Beatmungsschlauch mit einer durchgehenden Wand, die ein inneres Lumen begrenzt, in einem Extrusions- und Thermoformprozess über thermoplastische und -elastische Prozesse von einem Urform- in einen Umformprozess übergeführt, wobei das Lumen zur Leitung von Atemgas ausgebildet ist, wobei eine Helix die Wand auf der dem Lumen abgewandten Seite spiralförmig umgibt, wobei die Wand aus einem ersten Material hergestellt wird und wobei die Helix aus einem zweiten Material hergestellt wird, dadurch gekennzeichnet, dass die Helix mit der Wand verbunden ist.

Fig. 1

**Beschreibung**

Das Gebiet der vorliegenden Erfindung bezieht sich auf verbesserte Beatmungsschläuche.

Beatmungsschläuche sind ein wichtiger Bestandteil der medizinischen Beatmung. Die medizinische Beatmung wird bei Patienten mit Schwierigkeiten beim Atmen eingesetzt. Einige wichtige Faktoren, die bei Beatmungsschläuchen zu berücksichtigen sind: Beatmungsschläuche sollten flexibel sein, um sich den Bewegungen des Patienten anzupassen und die Intubation dadurch so angenehm wie möglich zu gestalten, weil bei Bewegungsänderungen oder ruckartige Bewegungen die entstehenden Krafteinwirkungen nicht unmittelbar bzw. unvermindert auf den Körper des Patienten einwirken. Die Größe des Beatmungsschlauchs ist bei einer invasiven, medizinischen Beatmung wichtig, um sicherzustellen, dass er zum Durchmesser der Atemwege des Patienten passt und eine ausreichende Luftzufuhr gewährleistet.

Beatmungsschläuche sollten aus einem Material hergestellt werden, das für den Patienten sicher ist und keine Allergien auslöst. Üblicherweise werden Schläuche beispielsweise aus TPE, Silikon oder PVC hergestellt. Die meisten Beatmungsschläuche bestehen aus flexiblem PVC-Material. Bei der Auswahl der Materialien müssen die Eigenschaften des Materials berücksichtigt werden, wie zum Beispiel die Flexibilität, die Festigkeit und die chemische Beständigkeit. Die PVC-Materialien werden durch ein Extrusionsverfahren in den gewünschten Schlauchdurchmesser und die Schlauchlänge gebracht. Dabei wird das Material durch eine Düse gepresst und auf eine Welle aufextrudiert, welche für die Innenformgebung und für das Wickeln und Formen des Schlauchs zuständig ist, sowie durch Kühlung zu einem festen Schlauch geformt. Eine gute Dichtigkeit des Beatmungsschlauchs ist entscheidend, um sicherzustellen, dass der Patient ausreichend Sauerstoff erhält und keine Luft entweicht. Beatmungsschläuche sollten über Anschlüsse verfügen, die es ermöglichen, sie sicher an das Beatmungsgerät oder die Atemmaske des Patienten anzuschließen. Die Länge des Schlauchs ist wichtig, um sicherzustellen, dass er lang genug ist, um bequem angeschlossen zu werden, aber nicht so lang, dass er unnötige Verwicklungen oder Verdrehungen verursacht, welche die erforderliche Sauerstoffzufuhr drosseln oder gar unterbrechen könnten. Beatmungsschläuche können steril sein, um Infektionen beim Patienten zu vermeiden. Beatmungsschläuche sind in der Regel jedoch nicht steril, was die Herstellungskosten senkt. Zudem sollten Beatmungsschläuche gut markiert sein, um die Positionierung im Körper des Patienten zu erleichtern und eine sichere Anwendung zu gewährleisten. Schläuche nach dem Stand der Technik sind in bestimmten Grenzen flexibel und nachgiebig.

Demgegenüber ist es die Aufgabe der vorliegenden Erfindung, einen Schlauch für die medizinische Beatmung zu verbessern. Insbesondere soll dieser komfortabler und zugleich zuverlässiger sein.

Diese Aufgabe wird mit einem Schlauch des Anspruchs 1 sowie mit einem Verfahren gemäß Anspruch 16 gelöst. Weiterbildungen und vorteilhafte Ausgestaltungen sind Gegenstand der Unteransprüche. Weitere Vorteile und Merkmale ergeben sich aus der allgemeinen Beschreibung und der Beschreibung der Ausführungsbeispiele. Während der Schlauch der vorliegenden Erfindung flexibel ist, ist er wünschenswerterweise auch weniger nachgiebig als Versionen des Standes der Technik. Vergleicht man einen Schlauch des Standes der Technik mit dem Schlauch der vorliegenden Erfindung, zeigt sich, dass der Schlauch des Standes der Technik sowohl axial als auch radial leichter expandiert wird.

Der erfindungsgemäße Schlauch bietet viele Vorteile. Einen erheblichen Vorteil bietet ein Beatmungsschlauch mit einer durchgehenden Wand, die ein inneres Lumen begrenzt, wobei das Lumen zur Leitung von Atemgas ausgebildet ist, wobei eine Helix die Wand, auf der dem Lumen abgewandten Seite spiralförmig umgibt, wobei die Wand aus einem ersten Material hergestellt ist und wobei die Helix aus einem zweiten Material hergestellt ist, wobei die Helix mit der Wand verbunden ist.

Die Helix und die Wand können auch aus unterschiedlichen Materialien hergestellt sein.

In allen Ausgestaltungen ist es besonders vorteilhaft und bevorzugt, dass die Helix eine Unterseite aufweist und mittels dieser unmittelbar und dauerhaft mit der Außenwand verbunden ist.

Es ist ebenfalls vorteilhaft und bevorzugt, dass die Helix im Querschnitt eine gerundete Außenkontur aufweist und sich von der Unterseite zu einer Oberseite verjüngt, in Richtung einer maximalen Helix-Höhe.

Die Helix-Höhe ist im Bereich 1,5 bis 3 mm, bevorzugt bei 2,3 mm, gemessen senkrecht von der Außenwand wegweisend.

In allen Ausgestaltungen ist es auch besonders vorteilhaft und bevorzugt, dass die Breite der Helix (an der Unterseite, die der Außenwand aufliegt) im Bereich 1,8 - 4 mm, bevorzugt 2,3 - 3,3 mm und besonders bevorzugt bei 2,9 mm +/- 0,4 mm liegt.

In einer vorteilhaften Ausgestaltung entspricht der Abstand zwischen zwei Helices der Steigung der Helix, wobei die Steigung bevorzugt, im Bereich 2 - 8 mm, bevorzugt auch im Bereich 3 - 7 mm, besonders bevorzugt bei 5 mm +/- 0,5 mm liegt.

Die Steigung wird direkt an der Außenwand, dort wo die Helix aufliegt, bestimmt.

Es ist ebenfalls vorteilhaft und bevorzugt, dass sich die Helix mit zunehmender Entfernung von der Außenwand verjüngt, wodurch der Abstand zwischen zwei benachbarten Helices zueinander an zumindest drei Stellen unterschiedlich ist und wobei der Abstand zwischen zwei benachbarten Helices im Bereich der maximalen Helix-Höhe am größten ist.

In allen Ausgestaltungen ist es besonders vorteilhaft und bevorzugt, dass die Leckage des Schlauches bei einem Atemgasdruck von 60 hPa kleiner 15 ml/min/m, bevorzugt kleiner 12 ml/min/m und besonders bevorzugt kleiner 10 ml/min/m ist. Eine Leckage im Beatmungsschlauch kann sehr gefährlich sein, da sie die Wirksamkeit der Beatmung beeinträchtigt und zu einer unzureichenden Atemgasversorgung des Patienten führen kann. Wenn eine Leckage im Beatmungsschlauch vermutet wird, sollte der Schlauch sofort ausgetauscht werden. Vor dem Austausch des Schlauchs sollte der Patient jedoch auf eine alternative Atemgasversorgung, wie z.B. eine manuelle Beatmung, umgestellt werden, um sicherzustellen, dass der Patient weiterhin ausreichend mit Sauerstoff versorgt wird.

Es ist auch wichtig, regelmäßig die Schläuche und alle anderen Beatmungsgeräte auf mögliche Leckagen oder andere Probleme zu überprüfen, um die Sicherheit des Patienten zu gewährleisten. Eine Schlauchleckage bezieht sich auf eine undichte Stelle oder ein Loch in einem Schlauch, durch die das Atemgas austreten kann. Schlauchleckagen können verschiedene Ursachen haben, wie beispielsweise Abnutzung, Beschädigung durch scharfe Gegenstände, unzureichende Befestigung, Druckänderungen, Alterung des Materials und viele andere. Insbesondere kann jedoch schon ein ungeeignetes Material auch ohne eine Beschädigung eine solche Leckage aufweisen, so dass eine Beatmung mit einem Schlauch aus diesem nicht geeigneten Material die Versorgung des Patienten gefährdet.

Es ist ebenfalls vorteilhaft und bevorzugt, dass die Federrate im Bereich 25 N/m ist oder größer 25 N/m, bevorzugt größer 27 N/m und besonders bevorzugt größer 30 N/m ist.

Die Federrate bezieht sich auf die Steifigkeit oder Elastizität des Schlauchmaterials. Je höher die Federrate, desto steifer ist der Schlauch und desto schwieriger ist es, ihn zu biegen oder zu verformen. Eine niedrigere Federrate bedeutet eine höhere Flexibilität und eine einfachere Handhabung des Schlauchs. Die Wahl der richtigen Federrate hängt von den Anforderungen der Anwendung ab und kann von Hersteller zu Hersteller variieren. Ein Schlauch mit einer geringen Federrate ist in der Regel flexibler und lässt sich einfacher biegen als ein Schlauch mit einer höheren Federrate. Dadurch kann er besser an verschiedene Formen und Konturen angepasst werden, was in vielen Anwendungen von Vorteil sein kann.

Ein weiterer Vorteil einer geringen Federrate ist, dass der Schlauch in der Regel weniger anfällig für Risse und Beschädigungen ist, da er sich bei Druckänderungen und Bewegungen besser anpassen kann und somit weniger Spannungen auftreten.

Allerdings kann ein Schlauch mit einer zu geringen Federrate auch instabil und leicht verformbar werden, was je nach Anwendung zu Problemen führen kann. Es ist daher wichtig, die richtige Federrate für die jeweilige Anwendung zu wählen.

Die Federrate eines Beatmungsschlauchs bezieht sich auf die Steifigkeit oder Widerstandsfähigkeit der Helix innerhalb des Schlauchs. Eine höhere Federrate bedeutet, dass mehr Druck erforderlich ist, um den Schlauch auszudehnen, während eine niedrigere Federrate bedeutet, dass weniger Druck erforderlich ist. Die Wahl der richtigen Federrate hängt von verschiedenen Faktoren ab, wie z.B. dem Druck und Volumen des Gases, das durch den Schlauch fließt, sowie den Anforderungen an die Beatmung des Patienten.

Eine hohe Federrate bei einem Schlauch bedeutet typischerweise, wenn steifere Materialien verwendet werden, dass der Schlauch steifer und widerstandsfähiger gegenüber Verformungen ist.

Bei der vorliegenden Erfindung ist die Wanddicke der Membrane ähnlich dick, wie bei einem deutlich unflexibleren Schlauch. Die Flexibilität wird über die Geometrie der Helix erzeugt.

Dies kann verschiedene Vorteile haben, je nach Anwendung des Schlauchs:
Reduzierte Dehnung: Wenn der Schlauch unter Druck steht, dehnt er sich aus. Eine hohe Federrate bedeutet, dass der Schlauch sich weniger dehnt, was den Druckverlust minimiert und die Effizienz der medizinischen Beatmung erhöht.

Präzise Steuerung: Ein steifer Schlauch ermöglicht eine präzisere Steuerung von Atemgasen. Dies ist besonders wichtig für die Vorgabe bestimmter Drücke, Flüsse/Flussgeschwindigkeiten oder Volumina.

Schläuche mit hoher Federrate halten Belastungen länger stand und sind weniger anfällig für Materialermüdung. Dies kann die Lebensdauer des Schlauchs verlängern.

Ein steifer Schlauch kann schneller auf Änderungen des Gasstroms reagieren. Dies kann in Anwendungen von Vorteil sein, bei denen Patiententrigger für die Steuerung der Beatmung ausgewertet werden.

Es ist jedoch zu beachten, dass eine zu hohe Federrate auch zu Nachteilen führen kann, wie z.B. einer erhöhten Wahrscheinlichkeit von Rissen oder Brüchen des Schlauchs bei zu hoher Belastung.

Compliance und Federrate sind beide Eigenschaften eines Schlauchs, beschreiben jedoch unterschiedliche Aspekte.

Compliance ist ein Maß für die Fähigkeit des Schlauchs, sich unter einer gegebenen Belastung oder Druckveränderung zu verformen. Es ist das Gegenteil von Steifigkeit und wird in Einheiten von Volumen pro Druckeinheit ausgedrückt. Compliance kann als die Menge an Volumen betrachtet werden, die ein Schlauch pro Einheit angewendeten Drucks ändern kann. Ein Schlauch mit hoher Compliance verformt sich leicht, während ein Schlauch mit geringer Compliance der Verformung widersteht.

In einer vorteilhaften Ausgestaltung ist die Compliance des Schlauches unter 1,0 ml / hPa / m ist, bevorzugt unter 0,8 und besonders bevorzugt 0,7 oder unter 0,7.

Die Federrate hingegen ist ein Maß für den Widerstand des Schlauchs gegen Verformung unter einer gegebenen Last oder einem gegebenen Druck. Es ist ein Maß für die Kraft, die erforderlich ist, um den Schlauch um eine bestimmte Länge zu komprimieren oder zu dehnen. Die Federrate wird in Einheiten von Kraft pro Einheit Länge ausgedrückt. Ein Schlauch mit hoher Federrate erfordert mehr Kraft, um ihn zu verformen als ein Schlauch mit niedriger Federrate. Die Federrate berücksichtigt nur eine lineare Zug- oder Druckkraft, um den Schlauch zu stauchen oder zu dehnen. Bei der Compliance liegt der Druck im Inneren des Schlauchs überall an. Hier hat die Geometrie der Wand weniger Einfluss als die Steifigkeit und die Wandstärke.

Zusammenfassend lässt sich sagen, dass die Compliance angibt, wie viel sich ein Schlauch unter einem gegebenen Druck verformt, während die Federrate misst, wie viel Kraft erforderlich ist, um den Schlauch um eine bestimmte Länge zu verformen. Je größer die Federrate ist, desto kleiner ist die Compliance, weil die größere Starrheit des Schlauches zugleich weniger Verformung unter Druck zulässt.

Der Schlauch ist vorzugsweise dazu geeignet und ausgebildet, dass die Dehnbarkeit im Bereich 3-39% liegt, bevorzugt im Bereich 5-36% und besonders bevorzugt im Bereich 8-30%.

Die Dehnbarkeit von Beatmungsschläuchen variiert je nach Material und Hersteller. In der Regel werden Beatmungsschläuche aus flexiblen Materialien wie TPE, Silikon oder PVC hergestellt, um eine gewisse Dehnbarkeit und Flexibilität zu gewährleisten. Die Dehnbarkeit wird oft durch den sogenannten "Dehnungsfaktor" angegeben, der angibt, um wie viel Prozent sich der Schlauch dehnen lässt, bevor er reißt oder beschädigt wird.

Es ist jedoch wichtig zu beachten, dass die Dehnbarkeit von Beatmungsschläuchen nicht der einzige Faktor ist, der bei der Auswahl eines geeigneten Schlauchs berücksichtigt werden sollte. Auch andere Faktoren wie Größe, Form, Wandstärke, Materialqualität und Verträglichkeit mit Desinfektionsmitteln müssen berücksichtigt werden, um einen geeigneten Schlauch für die medizinische Beatmung zu wählen. Die Dehnbarkeit eines Beatmungsschlauchs hängt von verschiedenen Faktoren ab, wie zum Beispiel dem Material, aus dem der Schlauch hergestellt ist, der Größe des Schlauchs und der Art der Belastung, der er ausgesetzt ist. Generell sind Beatmungsschläuche aus flexiblen Materialien wie z.B. Silikon oder Polyurethan gefertigt, die eine gewisse Dehnbarkeit aufweisen, um eine maximale Luftzufuhr und -abfuhr zu gewährleisten. Die genaue Dehnbarkeit hängt jedoch von der spezifischen Konstruktion und Herstellung des Schlauchs ab.

In einer vorteilhaften Ausgestaltung ist die Wand aus einem ersten Material TPE oder Silikon oder PVC und die Helix aus einem zweiten Material TPE oder Silikon oder PVC hergestellt.

Es ist möglich und vorteilhaft, dass das erste Material und das zweite Material identisch sind. Bei TPE kann die Verwendung in unterschiedlichen Härtegraden erfolgen.

Erfindungsgemäß ist der Schlauch auch so ausgebildet, dass das Gewicht pro Länge bei unter 1000g/m liegt, bevorzugt im Bereich zwischen 500 und 1000 g/m, besonders bevorzugt im Bereich zwischen 550 und 800 g/m.

Der vorliegende Beatmungsschlauch ist auch für Säuglinge, Neonaten und Frühgeborene anwendbar. Die Atemfrequenz eines Säuglings ist sehr hoch, sodass der Atemzyklus oder die Zeitspanne von der Einatmungsphase bis zur Ausatmungsphase sehr kurz ist. Die Lungen eines Frühgeborenen sind klein und steif und erfordern eine noch höhere Atemfrequenz, d. h. einen kürzeren, schnelleren Zyklus. Folglich ist während der Inspirations- oder Inhalationsphase eine hohe Flussrate erforderlich, um das gewünschte Atemgasvolumen innerhalb dieses kurzen Zyklus zu den Lungen des Säuglings zu übertragen. Je höher der Spitzendruck ist, desto höher ist die Durchflussrate, die erforderlich ist, um diesen Spitzendruck zu erreichen. Mit zunehmender Flussrate steigt auch der Ausatmungswiderstand (expiratorischer Gegendruck). Während der Ausatemphase muss der Patient Atemgas ausatmen. Es ist erwünscht, den Gegendruck innerhalb des Schlauches zu minimieren, damit der Patient diesen Druck beim Ausatmen nicht kraftraubend überwinden muss (expiratorischer Widerstand). Es ist daher wünschenswert, die Quellen sowohl des exspiratorischen Gegendrucks als auch des exspiratorischen Widerstands zu minimieren.

Durch die einzigartige Kombination des dünnwandigen Schlauchs und der Stützstruktur wird eine hohe Flexibilität der erfindungsgemäßen Schläuche erreicht.

Diese sind beispielhaft aus POLYVINYLCHLORID (PVC) oder POLYETHYLEN (PE) oder POLYPROPYLEN (PP) oder POLYESTER oder Silikon oder TPE hergestellt.

Die Erfindung umfasst auch ein Verfahren zur Herstellung eines Beatmungsschlauches mit einer durchgehenden Wand die ein inneres Lumen begrenzt in einem Extrusionsprozess, wobei das Lumen zur Leitung von Atemgas ausgebildet ist, wobei eine Helix die Wand auf der dem Lumen abgewandten Seite spiralförmig umgibt, wobei die Wand aus einem ersten Material hergestellt wird und wobei die Helix aus einem zweiten Material hergestellt wird dadurch gekennzeichnet, dass die Helix mit der Wand verbunden ist. Helix und Wand werden in einem Co-Extrusionsprozess hergestellt.

Die Helix ist notwendig, um dem Schlauch eine gewisse Stabilität zu verleihen, z.B. gegen äußere Krafteinwirkung. Bevorzugt sind Helix und Wand aus demselben Material.

Der erste Schritt des Verfahrens ist die Co-Extrusion des Ausgangsschlauches. Danach erfolgen die nachfolgend beschriebenen Verfahrensschritte, um den Schlauch flexibler und dehnbarer zu machen.

Bei dem Verfahren wird bevorzugt in einem Verfahrensschritt der Schlauch über die Erweichungstemperatur des Materials erwärmt und in einem zweiten Verfahrensschritt der Schlauch bezogen auf seine ursprüngliche Länge gestaucht und in einem dritten Verfahrensschritt der Schlauch abgekühlt, bis die Erstarrungstemperatur des Materials unterschritten ist.

Bei dem Verfahren erfolgen die Erwärmung und Stauchung unmittelbar nacheinander, bevor der gestauchte Schlauch abgekühlt wird.

Bei dem Verfahren wird der Schlauch auf über 70°C, bevorzugt auf über 80°C erwärmt und auf einem Block mechanisch gestaucht, sodass die Helices einander berühren. Dann wird der Schlauch auf eine Temperatur von unter 80°C, bevorzugt unter 70°C gekühlt, bevor die Stauchung beendet wird.

Der Schlauch wird bevorzugt von innen erwärmt und von innen gekühlt, beispielsweise durch einen Gasstrom.

Im Rahmen dieser Patentanmeldung werden die Begriffe Schlauch und Beatmungsschlauch synonym verwendet.

Die erfindungsgemäßen Schläuche stellen durch die folgenden Vorteile eine optimale Beatmung sicher: Ein transparenter Schlauch macht eine ständige Überwachung des Schlauchystems möglich. Eine verstärkende äußere Helix hält den Schlauchdurchmesser konstant, selbst wenn dieser gebogen ist. Der Schlauch ist somit flexibel und knickfest, sodass das Risiko einer Beatmungsverringerung oder -unterbrechung reduziert ist. Eine sehr niedrige Compliance stellt eine komfortable Anwendung sicher. Zugleiche weiche, leckagesichere und flexible Anschlüsse stellen eine perfekte Abdichtung sicher. Die Konnektoren sind direkt in den Schlauch integriert, um dichte Verbindungen sicherzustellen und Undichtigkeiten zu vermeiden. Die Schläuche können durch die Dehnbarkeit in der Länge ausgezogen werden.

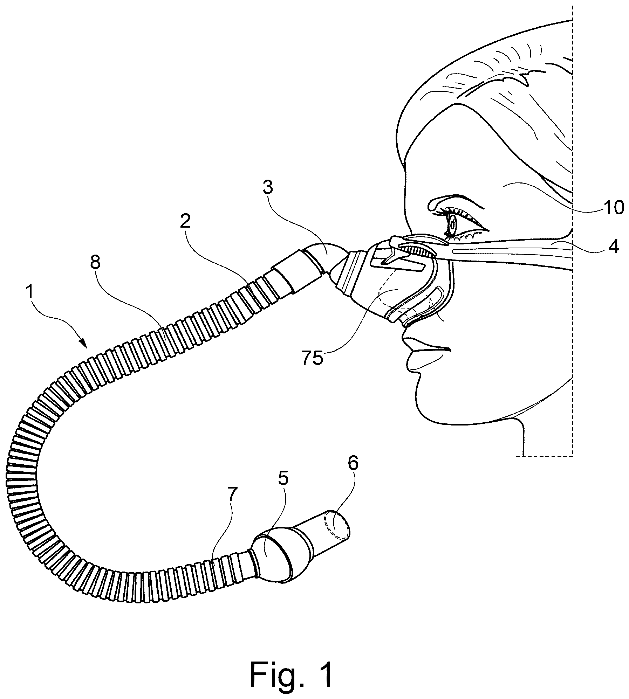

In Figur 1 ist der erfindungsgemäße Schlauch 1 dargestellt. Der Schlauch ist hier über ein Patienteninterface 75 und eine Bänderung 4 an einen Patienten 10 konnektiert. Die Verbindung zwischen Schlauch 1 und Patienteninterface 75 erfolgt über einen Maskenverbinder 3, die hier gekrümmt und mit einem Kugelgelenk ausgeführt ist. Der Maskenverbinder 3 wird über das Schlauchende 2 geschoben oder in das Schlauchende 2 eingeführt. Das andere Schlauchende ist mit einem Schlauchverbinder 5 verbunden. Der Schlauchverbinder 5 ist hier mit einem Kugelgelenk ausgeführt und weist an seinem dem Schlauch 1 abgewandten Ende einen Stutzen 6 auf, der der Verbindung mit einem anderen Beatmungsschlauch dient, der beispielsweise einen größeren Durchmesser als der Schlauch 1 aufweist. Alternativ kann das andere Schlauchende 7 mit einem nicht gezeigten Stutzen verbunden werden, der der direkten Konnektierung an ein Beatmungsgerät dient.

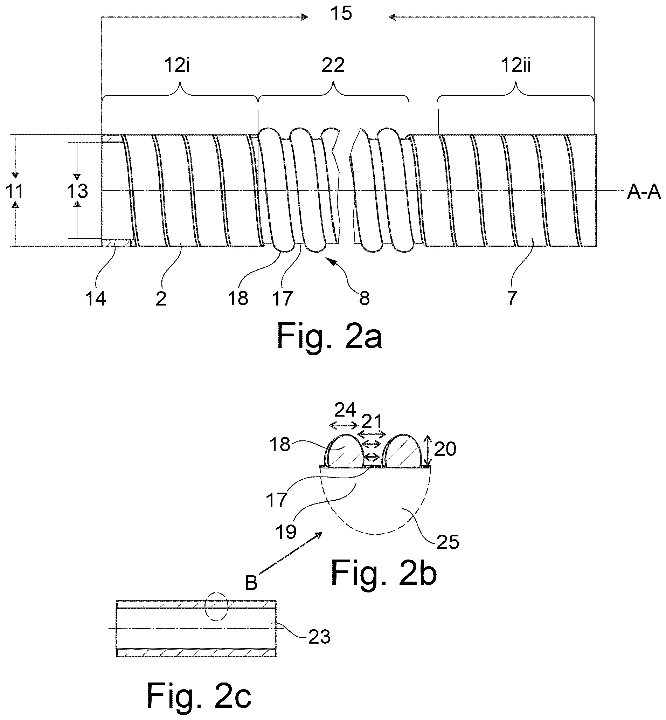

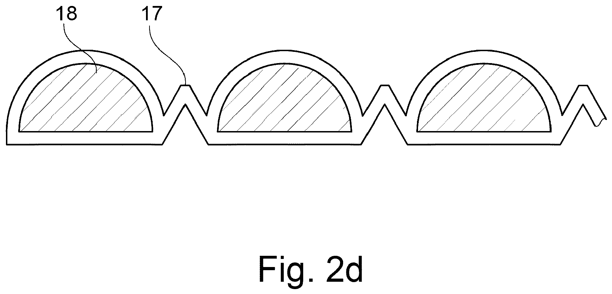

In Figur 2 ist der erfindungsgemäße Beatmungsschlauch 1 beispielhaft mit einigen Merkmalen dargestellt. Der Schlauch hat eine Länge 15, die beispielsweise bei 380 bis 400 mm liegt, bevorzugt bei 390 mm. Die Länge kann auch andere Werte annehmen, beispielsweise kann der Schlauch eine Länge von 1000 mm oder 2000 mm oder auch 3000 mm oder Zwischenwerten davon aufweisen. Die Länge 15 setzt sich zusammen aus den beiden Schlauchenden 2, 7 und dem Schlauchteil 8. Die Schlauchenden 2, 7 sind hier gleichlang dargestellt, sie können jedoch auch unterschiedliche Längen, Dicken und Formen aufweisen. Die Schlauchenden 2, 7 sind hier bevorzugt 10 - 30 mm lang, besonders bevorzugt 20 mm +/-4mm. Das Schlauchteil 8 macht üblicherweise den überwiegenden Teil der Gesamtlänge 15 aus, vorliegend ist das Schlauchteil 8 ohne die Schlauchenden etwa 350 mm lang.

Wie in den Fig.1 und 2 gezeigt, ist der Schlauch ein relativ dünnwandiger und flexibler Schlauch 1 mit einer äußeren Stützstruktur 18, die als spiralförmige Helix 18 ausgeführt ist, die über die Länge 15 des Schlauchs entlang seiner Außenwand 17 verläuft. Die Stützstruktur 18 verleiht dem Schlauch 1 strukturelle Festigkeit und Steifheit, während sie zudem eine Flexibilität bereitstellt. Die bereitgestellte strukturelle Festigkeit und Steifigkeit bietet Schutz vor Quetschungen durch äußere Kräfte, hilft, einen Druckkollaps zu vermeiden, und verringert die Nachgiebigkeit, die die Tendenz eines Schlauchs ist, sich bei Druckänderungen auszudehnen oder zusammenzuziehen. Obwohl die Stützstruktur 18 vorzugsweise spiralförmig ist oder spiralförmig um den Schlauch herum angeordnet ist, sind andere Formen möglich.

Fig. 2c zeigt einen Schnitt durch den Schlauch entlang der Linie A-A, teilweise mit Blick auf das Schlauchinnere. Die Innenfläche 23 des Schlauches kann glatt sein, was dazu beiträgt, sowohl den Inspirations- als auch den Exspirations-strömungswiderstand zu minimieren. Die Außenfläche des Schlauches ist geprägt durch die Helix 18, die mit der Außenwand 17 verbunden ist.

Fig. 2b zeigt einen vergrößerten Ausschnitt der Fig. 2c. Die Helix 18 ist mit einer Unterseite unmittelbar mit der Außenwand 17 (dauerhaft) verbunden. Die Helix 18 erstreckt sich von der Unterseite zur Oberseite gerundet und verjüngt sich somit in Richtung der maximalen Helix-Höhe 20. Die Breite 24 der Helix ist (an der Unterseite, die der Außenwand aufliegt) im Bereich 1,8 - 4 mm, bevorzugt 2,3 - 3,3 mm und besonders bevorzugt bei 2,9 mm +/- 0,4 mm. Die Helix-Höhe 20 ist im Bereich 1,5 bis 3 mm, bevorzugt bei 2,3 mm gemessen senkrecht von der Außenwand 17 wegweisend.

Der Abstand zwischen zwei Helices 18 entspricht der Steigung 21. Die Steigung ist bevorzugt im Bereich 3 - 8 mm, besonders bevorzugt liegt sie bei 5 mm +/- 0,5 mm. Die Steigung 21 wird direkt an der Außenwand 17, dort wo die Helix aufliegt, bestimmt. Dadurch, dass sich die Helix mit zunehmender Entfernung von der Außenwand 17 verjüngt, ist der Abstand zwischen zwei Helices 18 zueinander an zumindest drei Stellen unterschiedlich. Die sich verjüngende Breite 24 der Helix 18 trägt dazu bei, dass der Schlauch insgesamt biegsamer ist, da sich bei starker Biegung, die nach außen weisenden Bereiche der Helix erst spät berühren.

Fig. 2d zeigt ebenfalls einen vergrößerten Ausschnitt der Fig. 2c. Die Helix 18 ist mit einer Unterseite unmittelbar mit der Außenwand 17 verbunden. Bedingt durch den Stauchungsprozess ist die Wand zwischen den Helices Ziehharmonika-artig gefaltet. Die strukturelle Basis für den Schlauch bietet hierbei insbesondere die Helix.

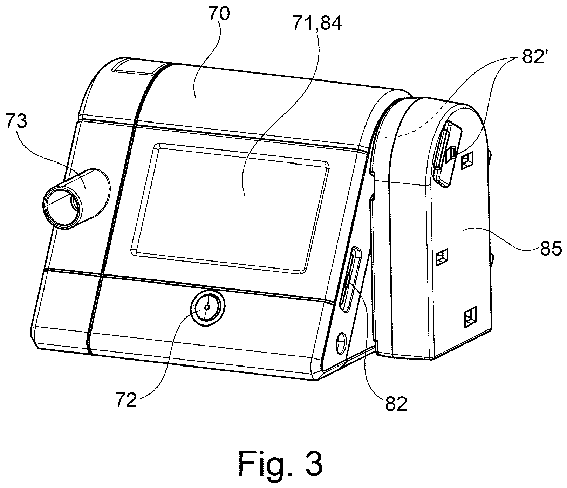

Fig.3 zeigt den grundsätzlichen Aufbau eines Beatmungsgerätes 70, welches mit einer Anzeigeeinrichtung 71, mindestens einem Bedienelement 72 sowie einem Schlauchanschluss 73 versehen ist. An den Schlauchanschluss 73 wird typischerweise über einen Beatmungsschlauch 74 ein Patienteninterface (beispielsweise als Atemmaske ausgebildet) 75 angeschlossen. Das Patienteninterface 75 und der Beatmungsschlauch 1 zur Atemgasversorgung eines Patienten sind nicht dargestellt. Das Beatmungsgerät 70 weist eine innenliegende Atemgasquelle 76, typischerweise in Form eines Gebläses, sowie eine Steuerung 77 dieser Atemgasquelle 76 auf. Zusätzlich weist das Beatmungsgerät 70 auch eine Messeinheit 78 sowie eine Speichereinheit 80 auf. Die Anzeigeeinrichtung 71 dient neben der Anzeige auch als Bedieneinheit 84 einer Mensch-Maschine-Schnittstelle, typischerweise in Form eines Touchscreen-Displays. Das Beatmungsgerät 70 ist typischerweise mit mindestens einer Kommunikationsschnittstelle 82 versehen. Die Schnittstelle 82 ist hier in einem Seitenbereich angeordnet. Die Schnittstelle 82 ist zur Horizontalen geneigt und befindet sich in räumlicher Nähe zur Anzeigeeinrichtung 71. Bevorzugt weist das Beatmungsgerät 70 zudem eine weitere Schnittstelle 82 auf, die der Kopplung von Modulen 85 dient. Messeinheiten in oder an dem Beatmungsgerät und/oder Beatmungsschlauch und/oder Patienteninterface sind für die Überwachung und Steuerung von Fluss und Druck vorgesehen, so dass der Druck und Fluss von Atemgas überwacht und erforderlichenfalls angepasst werden kann, auch unter Berücksichtigung der Einflüsse von Beatmungsschlauch und/oder Patienteninterface auf die Beatmung.

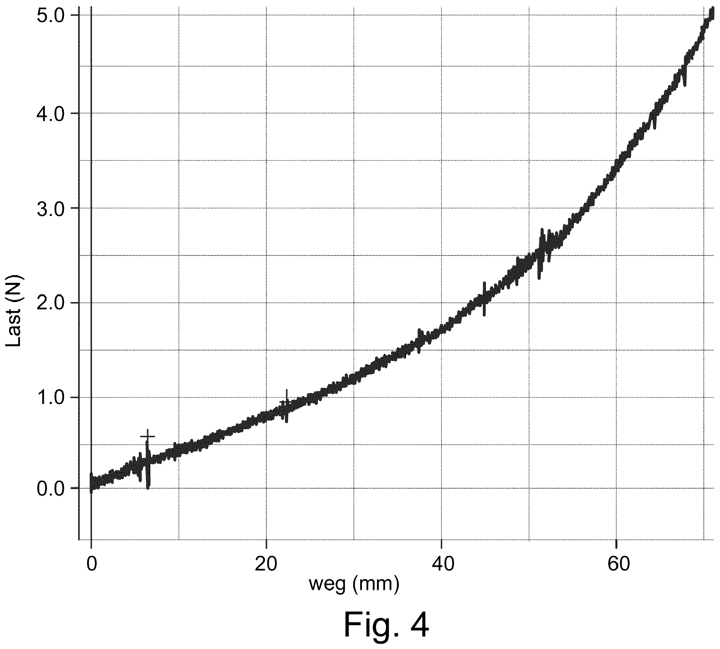

Der Graph von FIG. 4 zeigt die Federrate und die Dehnung eines beispielhaften Schlauches.

Berechnung Dehnung:

Die Ermittlung erfolgt bevorzugt dort, wo die nicht lineare Veränderung des Graphen anzeigt wird. Der Graph steigt dort senkrechter an (deutliche Erhöhung der Last bei gleichem Weg). An diesem Punkt liegt die maximale Dehnung des Schlauches durch die Helix. Wenn der Schlauch über diese Streckgrenze gedehnt wird, kommt er nicht mehr in seine Ausgangslänge zurück.

Die Dehnung beträgt hier also x mm.

Bei einer Ausgangslänge des Schlauches von y mm

Ergibt sich beispielsweise eine prozentuale Dehnung von: 15 - 30 %.

Die Berechnung der Federrate erfolgt mittels folgender Formel:

$$R = \frac{F}{\delta L}$$

F = die ermittelte Kraft in Newton (N) abgelesen vom Diagramm δL = Der zurückgelegte Weg bei der gewählten Kraft gemäß Diagramm

Hier ergibt sich:

Umrechnung δL von mm in m

$$z.B. \quad \frac{20\ mm}{1000 mm/m} = 0,02 \ m$$

Die Ermittlung der Kraft bei dem gewählten Weg von bspw. 20 mm erfolgt anhand des Diagramms. F = 0,75 N

Berechnung der Helixkonstante:

$$R = \frac{F}{\delta L} = \frac{0,75 N}{0,02 m} = 37,5 \ N/m$$

Die vorliegende Erfindung berücksichtigt, dass die Nachgiebigkeit, das Ausmaß der Ausdehnung der Schläuche während eines Atmungszyklus, eine wichtige Konstruktionsüberlegung ist. Der Grundprozess wird nun beschrieben. Der Druck und die Flussrate durch den Schlauch bleiben während des Atemzyklus im Wesentlichen gleich. Um den Patienten zu beatmen, wird während des Ausatmens ein Ventil am Beatmungsgerät geöffnet, wodurch der Patient Atemgas in den Schlauch ausstoßen kann. Dieser intermittierende Druckanstieg übt Expansionskräfte auf den Schlauch aus. Während des Einatmungsabschnitts des Atmungszyklus muss ein gewünschter Spitzendruck an den Lungen des Patienten erreicht werden, um ein gewünschtes Atemgasvolumen innerhalb der Einatmungszeitspanne abzugeben.

Je mehr sich der Schlauch ausdehnt, desto mehr Atemgas muss während der Inhalationsperiode geliefert werden, um den Spitzendruck zu erreichen, damit das gewünschte Atemgasvolumen an die Lunge des Patienten geliefert wird. Daher ist eine geringere Compliance des Schlauches wünschenswert.

Schläuche mit geringerer Nachgiebigkeit erfordern eine geringere Flussrate, um den gewünschten Spitzendruck zu erreichen. Da die Flussrate während der Inspirations- und Exspirationsphase konstant bleibt, haben Schläuche mit geringerer Compliance während der Exspirationsphase eine geringere Flussrate. Diese niedrigere Strömungsrate führt wünschenswerterweise zu einem niedrigeren Gegendruck, gegen den der Patient ausatmen muss. Folglich reduziert die überlegene niedrige Nachgiebigkeit der vorliegenden Erfindung den Gegendruck in genau dem optimalen Maße.

Die vorteilhaften Eigenschaften von Schläuchen gemäß der vorliegenden Erfindung lassen sich im Fertigungsprozess durch die Wahl von Material und Fertigungsbedingungen einstellen. Alternativ können erfindungsgemäß auch Schläuche zunächst fertiggestellt und nachträglich so modifiziert werden, dass die vorteilhaften Eigenschaften erzielt werden.

Vorversuche haben gezeigt, dass das folgende Vorgehen prinzipiell geeignet ist:

- Schlauch (von innen) erwärmen
- Schlauch stauchen
- Schlauch kühlen

Der Schlauch muss, um eine bleibende Verformung der Wand zu erreichen, über die Erweichungstemperatur des Materials erwärmt werden. Diese ist bei dem verwendeten Kunststoff TPE etwa 70 - 95°C, bevorzugt 85°C.

Die Herstellung erfolgt beispielsweise in einer Abfolge von Schritten;

Schlauch stauchen

Der erwärmte Schlauch muss gestaucht werden, damit die Eigenschaften geändert werden und erhalten bleiben.

Der Schlauch wird dazu "auf Block" gestaucht. Dabei grenzen die Helices aneinander und begrenzen so die Stauchung.

Sobald der Schlauch abgekühlt ist, kann die den Schlauch stauchende Kraft weggenommen werden.

Schlauch abkühlen

Sobald die Temperatur des Schlauches unterhalb der Erstarrungstemperatur des Materials ist, bleiben die - im Warmen eingebrachten - Verformungen bestehen. Dies ist bei dem verwendeten Kunststoff eine Temperatur von unter 85°C, bevorzugt unter 80°C oder sogar unter 70°C.

**Patentansprüche**

1. Beatmungsschlauch (1) mit einer durchgehenden Wand (17) die ein inneres Lumen (25) begrenzt, wobei das Lumen zur Leitung von Atemgas ausgebildet ist, wobei eine Helix (18) die Wand (17) auf der dem Lumen (25) abgewandten Seite spiralförmig umgibt, wobei die Wand (17) aus einem ersten Material hergestellt ist und wobei die Helix (18) aus einem zweiten Material hergestellt ist **dadurch gekennzeichnet, dass** die Helix (18) mit der Wand (17) verbunden ist.

2. Beatmungsschlauch (1) nach zumindest einem Anspruch 1 **dadurch gekennzeichnet, dass** die Helix (18) eine Unterseite aufweist und mittels dieser unmittelbar und dauerhaft mit der Außenwand (17) verbunden ist.

3. Beatmungsschlauch (1) nach zumindest einem der vorhergehenden Ansprüche **dadurch gekennzeichnet, dass** die Helix (18) im Querschnitt eine gerundete Außenkontur aufweist und sich von der Unterseite zu einer Oberseite verjüngt in Richtung einer maximalen Helix-Höhe (20).

4. Beatmungsschlauch (1) nach zumindest einem der vorhergehenden Ansprüche **dadurch gekennzeichnet, dass** die Breite (24) der Helix (18) (an der Unterseite, die der Außenwand aufliegt) im Bereich 1,8 - 4 mm, bevorzugt 2,3 - 3,3 mm und besonders bevorzugt bei 2,9 mm +/- 0,4 mm liegt.

5. Beatmungsschlauch (1) nach zumindest einem der vorhergehenden Ansprüche **dadurch gekennzeichnet, dass** der Abstand zwischen zwei Helices (18) der Steigung (21) der Helix entspricht, wobei die Steigung (21) bevorzugt im Bereich 3 - 8 mm, besonders bevorzugt bei 5 mm +/- 0,5 mm liegt.

6. Beatmungsschlauch (1) nach zumindest einem der vorhergehenden Ansprüche **dadurch gekennzeichnet, dass** sich die Helix (18) mit zunehmender Entfernung von der Außenwand (17) verjüngt, wodurch der Abstand zwischen zwei benachbarten Helices (18) zueinander an zumindest drei Stellen unterschiedlich ist und wobei der Abstand zwischen zwei benachbarten Helices (18) im Bereich der maximalen Helix-Höhe (20) am größten ist.

7. Beatmungsschlauch (1) nach zumindest einem der vorhergehenden Ansprüche **dadurch gekennzeichnet, dass** die die Helix (18) die Wand (17) in Form einer Helix umgibt 2,7 mm mit 5 mm Steigung bei einem Innendurchmesser von 015 mm Schlauch.

8. Beatmungsschlauch (1) nach zumindest einem der vorhergehenden Ansprüche **dadurch gekennzeichnet, dass** die die Wand () eine Dicke von 0,05 bis 0,3 mm, bevorzugt 0,1 bis 0,2 mm aufweist.

9. Beatmungsschlauch (1) nach zumindest einem der vorhergehenden Ansprüche **dadurch gekennzeichnet, dass** die Compliance des Schlauches unter 1,2 ml / hPa / m ist, bevorzugt unter 1,0 und besonders bevorzugt unter 0,8 oder auch unter 0,7.

10. Beatmungsschlauch (1) nach zumindest einem der vorhergehenden Ansprüche **dadurch gekennzeichnet, dass** die Leckage bei einem Atemgasdruck von bis zu 60 hPa kleiner 15 ml/min/m, bevorzugt kleiner 12 ml/min/m und besonders bevorzugt kleiner 10 ml/min/m ist.

**11.** Beatmungsschlauch (1) nach zumindest einem der vorhergehenden Ansprüche **dadurch gekennzeichnet, dass** die Federrate im Bereich 25 N/m ist oder größer 25 N/m, bevorzugt größer 28 N/m und besonders bevorzugt größer 31 N/m ist.

**12.** Beatmungsschlauch (1) nach zumindest einem der vorhergehenden Ansprüche **dadurch gekennzeichnet, dass** die Dehnbarkeit im Bereich 3-38% liegt, bevorzugt im Bereich 5-34% und besonders bevorzugt im Bereich 8-30%

**13.** Beatmungsschlauch (1) nach zumindest einem der vorhergehenden Ansprüche **dadurch gekennzeichnet, dass** die Wand aus einem ersten Material Silikon hergestellt ist und wobei die Helix (18) aus einem zweiten Material PVC oder TPE hergestellt ist.

**14.** Beatmungsschlauch (1) nach zumindest einem der vorhergehenden Ansprüche **dadurch gekennzeichnet, dass** das erste Material und das zweite Material identisch sind.

**15.** Beatmungsschlauch (1) nach zumindest einem der vorhergehenden Ansprüche **dadurch gekennzeichnet, dass** das Gewicht pro Länge bei unter 1000g/m liegt, bevorzugt im Bereich zwischen 500 und 1000 g/m, besonders bevorzugt im Bereich zwischen 550 und 800 g/m.

**16.** Verfahren zur Herstellung eines Beatmungsschlauches (1) mit einer durchgehenden Wand (17), die ein inneres Lumen (25) begrenzt in einem Extrusionsprozess, wobei das Lumen (25) zur Leitung von Atemgas ausgebildet ist, wobei eine Helix (18) die Wand (17) auf der dem Lumen (25) abgewandten Seite spiralförmig umgibt, wobei die Wand (17) aus einem ersten Material hergestellt wird und wobei die Helix (18) aus einem zweiten Material hergestellt wird **dadurch gekennzeichnet, dass** die Helix (18) mit der Wand (17) verbunden ist und Helix und Wand in einem Co-Extrusionsprozess hergestellt werden.

**17.** Verfahren zur Herstellung eines Beatmungsschlauches (1) nach Anspruch 16, bei dem in einem ersten Verfahrensschritt der Schlauch über die Erweichungstemperatur des Materials erwärmt wird und in einem zweiten Verfahrensschritt der Schlauch bezogen auf seine ursprüngliche Länge gestaucht wird und in einem dritten Verfahrensschritt der Schlauch abgekühlt wird bis die Erstarrungstemperatur des Materials unterschritten ist.

**18.** Verfahren zur Herstellung eines Beatmungsschlauches (1) nach Anspruch 16 oder 17, bei dem die Erwärmung und Stauchung unmittelbar nacheinander erfolgen und bei dem der gestauchte Schlauch abgekühlt wird.

**19.** Verfahren zur Herstellung eines Beatmungsschlauches (1) nach zumindest einem der vorhergehenden Ansprüche bei dem der Schlauch auf über 70°C, bevorzugt auf über 80°C erwärmt wird und auf Block gestaucht wird, sodass die Helices einander berühren, und bei dem der Schlauch auf eine Temperatur von unter 80°C, bevorzugt unter 70°C gekühlt wird, bevor die Stauchung beendet wird.

**20.** Verfahren zur Herstellung eines Beatmungsschlauches (1) nach zumindest einem der vorhergehenden Ansprüche bei dem der Schlauch von innen erwärmt und von innen gekühlt wird.

Fig. 1

Fig. 2a

Fig. 2b

Fig. 2c

Fig. 2d

Fig. 3

Fig. 4

Europäisches Patentamt
European Patent Office
Office européen des brevets

# EUROPÄISCHER RECHERCHENBERICHT

**Nummer der Anmeldung**

EP 24 17 0740

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2014/130931 A1 (FORRESTER MARTIN E [CA]) 15. Mai 2014 (2014-05-15) * Abbildungen 1, 2, 4 * * Absätze [0006], [0020], [0021], [0045] - [0048] * | 1-3,6, 14,16-20 | INV. A61M16/08 F16L11/04 |
| | ----- | | |
| X | US 6 571 794 B1 (HANSEN GARY [US]) 3. Juni 2003 (2003-06-03) * Spalte 3, Zeile 32 - Spalte 4, Zeile 6; Abbildungen 1, 3 * | 1-3,14, 16 | |
| | ----- | | |
| X | US 2014/000626 A1 (O'CONNOR MARK THOMAS [NZ] ET AL) 2. Januar 2014 (2014-01-02) * Abbildungen 3b, 3e * * Absätze [0470], [0475], [0491] - [0493], [0510], [0518], [0519], [0535] * | 1-5,7-16 | |
| | ----- | | |
| X | DE 10 2009 009790 B3 (SCHAUENBURG HOSE TECHNOLOGY GM [DE]) 17. Juni 2010 (2010-06-17) * Abbildungen 1-3 * * Absätze [0001], [0010] * | 1-3,6 | |
| | ----- | | RECHERCHIERTE SACHGEBIETE (IPC) |
| X | WO 2022/246520 A1 (RESMED PTY LTD [AU]) 1. Dezember 2022 (2022-12-01) * Absätze [0034], [0041], [0118]; Abbildung 8 * | 1 | A61M |
| | ----- | | |

Der vorliegende Recherchenbericht wurde für alle Patentansprüche erstellt

| Recherchenort | Abschlußdatum der Recherche | Prüfer |
|---|---|---|
| München | 17. September 2024 | Trattner, Barbara |

**ANHANG ZUM EUROPÄISCHEN RECHERCHENBERICHT
ÜBER DIE EUROPÄISCHE PATENTANMELDUNG NR.**

EP 24 17 0740

In diesem Anhang sind die Mitglieder der Patentfamilien der im obengenannten europäischen Recherchenbericht angeführten
Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

17-09-2024

| Im Recherchenbericht angeführtes Patentdokument | Datum der Veröffentlichung | Mitglied(er) der Patentfamilie | | Datum der Veröffentlichung |
|---|---|---|---|---|
| US 2014130931 A1 | 15-05-2014 | KEINE | | |
| US 6571794 B1 | 03-06-2003 | US | 6571794 B1 | 03-06-2003 |
| | | WO | 0232492 A2 | 25-04-2002 |
| US 2014000626 A1 | 02-01-2014 | AU | 2011318681 A1 | 02-05-2013 |
| | | AU | 2016203303 A1 | 09-06-2016 |
| | | AU | 2016222390 A1 | 15-09-2016 |
| | | AU | 2018204295 A1 | 05-07-2018 |
| | | AU | 2020200958 A1 | 05-03-2020 |
| | | AU | 2021290239 A1 | 17-02-2022 |
| | | AU | 2024202722 A1 | 16-05-2024 |
| | | CA | 2814601 A1 | 26-04-2012 |
| | | CA | 3177429 A1 | 26-04-2012 |
| | | CN | 103249448 A | 14-08-2013 |
| | | CN | 105999506 A | 12-10-2016 |
| | | CN | 106310479 A | 11-01-2017 |
| | | CN | 107684651 A | 13-02-2018 |
| | | CN | 107684652 A | 13-02-2018 |
| | | CN | 107773824 A | 09-03-2018 |
| | | CN | 107773825 A | 09-03-2018 |
| | | CN | 113440711 A | 28-09-2021 |
| | | DE | 112011103502 T5 | 05-12-2013 |
| | | EP | 2629821 A1 | 28-08-2013 |
| | | EP | 3556416 A1 | 23-10-2019 |
| | | EP | 4218871 A2 | 02-08-2023 |
| | | ES | 2736776 T3 | 07-01-2020 |
| | | ES | 2971725 T3 | 06-06-2024 |
| | | GB | 2498144 A | 03-07-2013 |
| | | GB | 2527221 A | 16-12-2015 |
| | | GB | 2529076 A | 10-02-2016 |
| | | GB | 2531184 A | 13-04-2016 |
| | | GB | 2531185 A | 13-04-2016 |
| | | GB | 2531985 A | 04-05-2016 |
| | | GB | 2532170 A | 11-05-2016 |
| | | JP | 5997698 B2 | 28-09-2016 |
| | | JP | 6339140 B2 | 06-06-2018 |
| | | JP | 6559834 B2 | 14-08-2019 |
| | | JP | 6902575 B2 | 14-07-2021 |
| | | JP | 7546837 B2 | 09-09-2024 |
| | | JP | 2013540037 A | 31-10-2013 |
| | | JP | 2017018614 A | 26-01-2017 |
| | | JP | 2018153659 A | 04-10-2018 |
| | | JP | 2019213869 A | 19-12-2019 |
| | | JP | 2021166728 A | 21-10-2021 |
| | | JP | 2023088983 A | 27-06-2023 |

Für nähere Einzelheiten zu diesem Anhang : siehe Amtsblatt des Europäischen Patentamts, Nr.12/82

EPO FORM P0461

Seite 1 von 2

**ANHANG ZUM EUROPÄISCHEN RECHERCHENBERICHT
ÜBER DIE EUROPÄISCHE PATENTANMELDUNG NR.**

EP 24 17 0740

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

17-09-2024

| Im Recherchenbericht angeführtes Patentdokument | Datum der Veröffentlichung | Mitglied(er) der Patentfamilie | Datum der Veröffentlichung |
|---|---|---|---|
| | | PL 3556416 T3 | 22-04-2024 |
| | | US 2014000626 A1 | 02-01-2014 |
| | | US 2019217038 A1 | 18-07-2019 |
| | | US 2022362503 A1 | 17-11-2022 |
| | | WO 2012053910 A1 | 26-04-2012 |
| DE 102009009790 B3 | 17-06-2010 | DE 102009009790 B3 | 17-06-2010 |
| | | US 2010215351 A1 | 26-08-2010 |
| WO 2022246520 A1 | 01-12-2022 | EP 4346967 A1 | 10-04-2024 |
| | | US 2024238548 A1 | 18-07-2024 |
| | | WO 2022246520 A1 | 01-12-2022 |

Für nähere Einzelheiten zu diesem Anhang : siehe Amtsblatt des Europäischen Patentamts, Nr.12/82

EPO FORM P0461

Seite 2 von 2